Europäisches Patentamt

European Patent Office

Office européen des brevets

(19)

(11) Publication number: **0 102 642**
**B1**

(12) **EUROPEAN PATENT SPECIFICATION**

(45) Date of publication of patent specification: **29.01.86**

(21) Application number: **83108736.6**

(22) Date of filing: **05.09.83**

(51) Int. Cl.⁴: **C 07 C 57/04, C 07 C 51/487,**
**C 07 C 51/42, C 07 C 51/48**

(54) **Process for purifying methacrylic acid.**

(30) Priority: **06.09.82 JP 153818/82**

(43) Date of publication of application:
**14.03.84 Bulletin 84/11**

(45) Publication of the grant of the patent:
**29.01.86 Bulletin 86/05**

(84) Designated Contracting States:
**DE FR GB**

(56) References cited:
**EP-A-0 023 774**
**US-A-2 288 281**
**US-A-2 394 572**
**US-A-2 457 257**
**S.PATAI: "THE CHEMISTRY OF THE CABRONYL GROUP", 1966, PP. 388-390, INTERSCIENCE PUBLISHERS, LONDON (GB)**
**J.G.HANNA: "CHEMICAL AND PSYSICAL METHODS OF ANALYSIS"**

**The file contains technical information submitted after the application was filed and not included in this specification**

(73) Proprietor: **Nippon Shokubai Kagaku Kogyo Co., Ltd**
**1, 5-chome, Koraibashi Higashi-ku**
**Osaka (JP)**

(72) Inventor: **Shimizu, Noboru**
**11-20, Sakae-machi 2-chome**
**Takatsuku-shi Osaka-fu (JP)**
Inventor: **Daigo, Hiromiki**
**13-401, No. 2639, Aomadani**
**Minoo-shi Osaka-fu (JP)**
Inventor: **Yoshida, Hiroshi**
**21-7, Uenosaka 1-chome**
**Toyonaka-shi Osaka-fu (JP)**
Inventor: **Matumoto, Shoichi**
**3-3, Ayaba 2-chome**
**Ikeda-shi Osaka-fu (JP)**

(74) Representative: **Kraus, Walter, Dr. et al**
**Patentanwälte Kraus Weisert & Partner Thomas-Wimmer-Ring 15**
**D-8000 München 22 (DE)**

Courier Press, Leamington Spa, England.

## Description

This invention relates to a process for purifying methacrylic acid (to be sometimes abbreviated as MAA hereinafter). More specifically, this invention relates, in the production of MAA by the catalytic vapor-phase oxidation reaction of isobutylene, tertiary butanol, methacrolein or isobutyraldehyde, to a process for producting MAA of high purity and high quality from a crude MAA solution obtained from the oxidation reaction product gas with good industrial efficiency and without any process trouble.

It is known from many literature references on the development of oxidation catalysts or processes that MAA can be produced by catalytically oxidizing isobutylene, tertiary butanol, methacrolein or isobutyraldehyde in the vapor phase in one or more reaction stages. But considerably long years elapsed before MAA went into industrial production by catalytic vapor-phase oxidation reaction. The reason is that since various by-products exist in the reaction product gas and some of these by-products are carried over to the crude MAA solution, they cause various troubles in the process of purifying MAA or reduce the quality of the final MAA product. Specifically, the reaction product gas contains small amounts of relatively low-boiling substances such as methacrolein, acrolein, acrylic acid, acetic acid, acetaldehyde and carbon oxide, maleic acid, aromatic carboxylic acids such as terephthalic acid, toluic acid and benzoic acid, and tarry substances, in addition to the main product MAA. Among them, relatively high-boiling by-products (aromatic compounds and tarry substances) which dissolve in the crude MAA solution cause various troubles in the subsequent purification of MAA, and hamper the continuous operation of the process.

In order to reduce these troubles, various proposals have been made heretofore. For example, Japanese Laid-Open Patent Publication No. 52021/1975 proposes a method of preventing the generation of a scum by treating an aqueous solution of crude MAA with activated carbon or a special ion-exchange resin before its extraction, and the published specification of EP 23774A proposes a method of separating and removing a scum which deposits on the interface between a solvent layer and an aqueous layer during extraction. These methods, however, only complicate the process, and do not give a complete solution to the problem. According to these methods, the prevention of scum formation or its separation and removal cannot be fully achieved, and troubles still occur in long operations. Furthermore, much waste water is formed as a result of washing the apparatus. For this reason, these methods cannot be accepted industrially.

GB 2,045,759 specification proposes a method in which a mixture of at least two solvents is used as an extractant in order to remove the trouble attributed to the deposition of solids during operation in a distillation column. By using this method, the troubles which occur in the extracting and distilling operations are reduced only to some extent, and in a long continuous operation, no satisfactory result is obtained. In addition, this method requires complex operations for controlling the mixing ratio of the solvents or recovering the solvents.

US—A—2,394,572 describes a process for the decolorization of a reaction liquid by treating with bisulfite in the production of acrylic acids or methacrylic acids by liquid phase oxidation of acrolein or methacrolein in the presence of a phenolic polymerization inhibitor.

The present inventors made extensive investigations about the causes of the troubles which occur owing to the deposition of solids in an extracting column or a distillation column in the separation of MAA from a crude MAA solution containing these high-boiling by-products as impurities and its purification, and about a method of preventing the deposition of solids. These investigations have led to the discovery that by first adding a bisulfite to the crude MAA solution and then subjecting the mixture to subsequent process steps, the troubles in the process are drastically reduced, and MAA of high quality can be obtained.

Impurities which deposit as solids in the extraction column or the distillation column are presumed to be aldehydes or aldehyde derivatives having a high boiling point. These compounds form water-soluble adducts by the addition of the bisulfite, and become difficult to extract in the extracting operation. They are left in the extraction residue, and thus separated from the extracted MAA. This is the same for the case of an organic solvent solution of MAA. When an aqueous solution of a bisulfite is added to the organic solvent solution of MAA and the mixture is left to stand, the mixture separates into two layers, and the impurities do not easily migrate to the organic layer. Hence, a trouble does not appreciably occur in the subsequent distillation operation. When MAA does not substantially contain an organic solvent, the impurities can be removed from MAA by mixing MAA with a suitable amount of an organic solvent and an aqueous solution of a bisulfite to separate the mixture into two layers.

When the bisulfite is not added to an aqueous solution of crude MAA, extraction results in the transfer of MAA from the aqueous solution to the solvent and the concentration of MAA in the aqueous solution decreases. This naturally reduces the solubility of the impurities, and the impurities dissolved in the aqueous solution of crude MAA precipitate on the interface. Furthermore, when the bisulfite is not added to the aqueous solution of crude MAA, the amount of impurities composed mainly of aldehydes which have been extracted increases. Since these impurities are sent to the subsequent distillation step together with MAA and the solvent, polymerization is liable to occur during the distillation operation.

The present invention is based on the above discovery, and its basic principle is that high-boiling aldehydes which are among the by-products impurities and cause troubles in the extracting and distilling steps react with the bisulfite to form adducts having high water solubility and in extraction with an organic solvent, these adducts remain in the aqueous layer, and MAA migrates to the organic layer. As stated hereinabove, solids which precipitate on the interface at the time of extraction are certain high-boiling aldehydes. These aldehydes dissolve in the aqueous solution by the aid of MAA before the extraction, but as MAA is extracted and its concentration in the aqueous solution decreases, they precipitate. These high-boiling aldehydes are partly extracted with the organic solvent and act as a polymerization promoter in the subsequent distilling step. This trouble can be eliminated in accordance with the above principle by adding the bisulfite to the crude MAA solution.

According to this invention, the addition of the bisulfite makes it difficult to extract the by-product maleic acid, and the process of purifying MAA can be simplified.

Furthermore, according to this invention, protoanemonin, which is a by-product of oxidation reaction, can be removed effectively. Protoanemonin is formed as a by-product in an amount of about 200 to 1,000 ppm (based on MAA) in the catalytic vaporphase oxidation reaction of isobutylene, tertiary butanol, methacrolein or isobutyraldehyde. Even after performing extraction of MAA with ordinary solvents and steps of its separation and purification by distillation, protoanemonin tends to accompany MAA and causes a decrease in the quality of MAA. Protoanemonin can be separated from MAA by a distillation operation. But since the relative volatility of these is low, many trays and a high reflux ratio are required in order to decrease protoanemonin to a satisfactory amount, and this is not economical. When the bisulfite is added to the crude MAA solution, protoanemonin present in the solution combines with the bisulfite to form some adduct which is difficult to extract. Hence, the amount of protoanemonin distributed to the aqueous layer becomes larger than that distributed to the organic layer. As a result, the amount of protoanemonin extracted is small than when the bisulfite is not added. This operation, however, can reduce the concentration of protoanemonin in MAA only to 20 to 400 ppm (based on MAA), and the quality of MAA as a final product is not yet satisfactory.

The process of the present invention for purifying methacrylic acid comprises bringing a solution of crude methacrylic acid obtained from the reaction product gas of said oxidation into contact with an aqueous solution of a bisulfite in the presence of a substantially water-insoluble organic solvent to separate it into an organic layer and an aqueous layer, feeding the organic layer into a solvent separating column and distilling off the solvent mainly, feeding the distillation bottom of the solvent separating column into a low-boiling substance separating column and distilling off low-boiling substances, mixing the low-boiling substances with said crude methacrylic acid solution, and feeding the distillation bottom of the low-boiling substance separating column into a high-boiling substance separating column and recovering methacrylic acid of high purity as a distillate.

The embodiments of this invention are described below spefically.

The crude MAA solution used in this invention is either an aqueous solution of MAA usually in a concentration of 15 to 50% by weight obtained by pasing isobutylene, tetiary butanol, methacrylein or isobutyraldehyde through one or more catalyst layers to oxidize it catalytically in the vapor phase, rapidly cooling and condensing the reaction product gas to collect an aqueous solution containing MAA, and removing low-boiling aldehydes by stripping, or a solution of MAA usually in a concentration of at least 50% by weight obtained by subjecting the aforesaid MAA-containing aqueous solution to an extraction step and a distillation step. Accordingly, in addition to MAA, the crude MAA solution contains high-boiling aromatic carboxylic acids, high-boiling aldehydes, tarry substances, water, organic solvents, etc.

Examples of the bisulfite used in this invention are bisulfites of alkali metals such as Na, K and Cs and ammonium bisulfite, the sodium, potassium and ammonium bisulfites being preferred. Preferably, the bisulfite is added in the form of an aqueous solution having a bisulfite concentration of usually from 10% by weight to its saturation concentration. The amount of the bisulfite is 0.1 to 15% by weight, preferably 1 to 10% by weight, based on MAA in the crude MAA solution, and may vary depending upon the content of impurities in the crude MAA solution.

In the process of purifying MAA by adding the bisulfite to the crude MAA solution, the presence of a water-insoluble organic solvent is essential. It should be noted however that depending upon the composition of the crude MAA solution, the method of treatment with the organic solvent differs as described below.

When the crude MAA solution does not contain water and a greater portion of it is MAA, an aqueous solution of the bisulfite and an organic solvent are added to bring them into contact with the crude MAA solution. The mixture is then separated into two layers and the impurities in MAA are transferred to the aqueous layer. It is preferred to determine the amount of the solvent and the amount of water in the bisulfite solution so that a greater portion of MAA may be transferred to the solvent layer and the amount of MAA transferred to the aqueous layer may become not more than 10% by weight, preferably not more than 5% by weight, of MAA originally present. When the amount of the organic solvent is too small, the treated solution does not separate into two layers. Hence, the amount of the solvent is 10 to 50% by weight, preferably 20 to 40% by weight, based on the entire methacrylic solution. Accordingly, when an organic solvent is present in the crude MAA solution, the solvent may be added so that the total amount of it is within the above-mentioned range.

Mixing of the crude MAA solution with the aqueous bisulfite solution can be usually carried out batchwise or continuously by a mixer such as a line mixer or a stirred mixing tank. There can also be used a batch method which comprises optionally adding an organic solvent to the crude MAA solution, adding an aqueous solution of the bisulfite, mixing them with stirring, and leaving the mixture to stand and a method which comprises continuously introducing the three solutions into a mixer, continuously mixing them with stirring, feeding the mixture into a standing vessel, and withdrawing the oil layer and the aqueous layer continuously from the standing vessel. Alternatively, after adding the aqueous bisulfite solution to the crude MAA solution, the mixture can be contacted with an organic solvent by a continuous extracting operation. This method requires additional equipment, but can achieve efficient purification. The mixing temperature is ordinary temperature to 60°C. Methacrylic acid having high purity and high quality can be obtained by separating the solvent from the oily layer, separating low-boiling substances, and then removing high-boiling substances. The aqueous layer is discharged out of the system, or if required, methacrylic acid dissolved in the aqueous layer may be recovered by extraction with a solvent.

When the crude MAA solution is an aqueous solution of crude MAA, it is well mixed with an aqueous solution of the bisulfite. The mixture is introduced into an extraction column, and extracted in a usual manner with an organic solvent whereby an extract and an extraction residue are obtained. The extract still contains some impurities, for example high-boiling aldehydes and protoanemonin. These impurities can be removed well by washing the extract with an aqueous solution of the bisulfite. When this washing operation is carried out, the aqueous layer resulting after contacting the aqueous bisulfite solution with the extract is preferably used as a bisulfite source for the aqueous MAA solution. By subjecting the extract washed with the aqueous bisulfite solution to a step of separating the solvent, a step of separating low-boiling substances and a step of separating high-boiling substances, MAA of high purity can be obtained.

As an alternative, when the extract is not washed with the aqueous bisulfite solution, the extract is directly subjected to a step of separating the solvent and a step of separating low-boiling substances. Since protoanemonin remains in a concentration of 20 to 400 ppm based on MAA, it should be distilled in the step of separating low-boiling substances to a concentration of less than 20 ppm. Since the relative volatilities of protoanemonin and MAA are low, a large amount of MAA is included in the distillate together with protoanemonin during the separation of protoanemonin. This MAA should be recovered. To achieve this, all the distillate resulting in the step of separating low-boiling substances is mixed with the aqueous MAA solution before extraction and then the bisulfite is added. As a result, protoanemonin and aldehydes remain in the aqueous layer, and MAA is extracted by the solvent. Thus, MAA can be recovered and the impurities such as protoanemonin can be efficiently removed out of the system. Another method for mixing the distillate with the aqueous crude MAA solution before extraction comrpises contacting the distillate directly with the bisulfate and feeding the mixture to the extracting step, or directly feeding the distillate to the exctraction step without contacting. These alternative methods can be employed as required. The proportion of the bisulfite to be added to the distillate of the low-boiling substance separating column is determined depending upon the weight of MAA in the distillate as in the aforesaid cases.

In the present invention, known organic solvents can be used. Examples include aromatic compounds such as benzene, toluene, xylenes and ethylbenzene; esters such as isopropyl acetate, isobutyl acetate, n-butyl acetate and methyl methacrylate; hydrocarbons such as n-hexane and n-heptane; and ketones such as isophorone and methyl isobutyl ketone.

The process of this invention is carried out in the presence of a known conventional polymerization inhibitor, such as hydroquinone, methoxyhydroquinone monomethyl ether, methylene blue, phenothiazine, copper salicylate, copper dialkyldithiocarbamates, and molecular oxygen.

MAA purified by the process of this invention has high purity and good polymerization characteristics, and has a protoanemonin content of less than 20 ppm. According to this invention, MAA purified to a high degree can be obtained industrially advantageously without any trouble in the purification process.

The present invention is described more specifically with reference to flow sheets shown in the accompanying drawings.

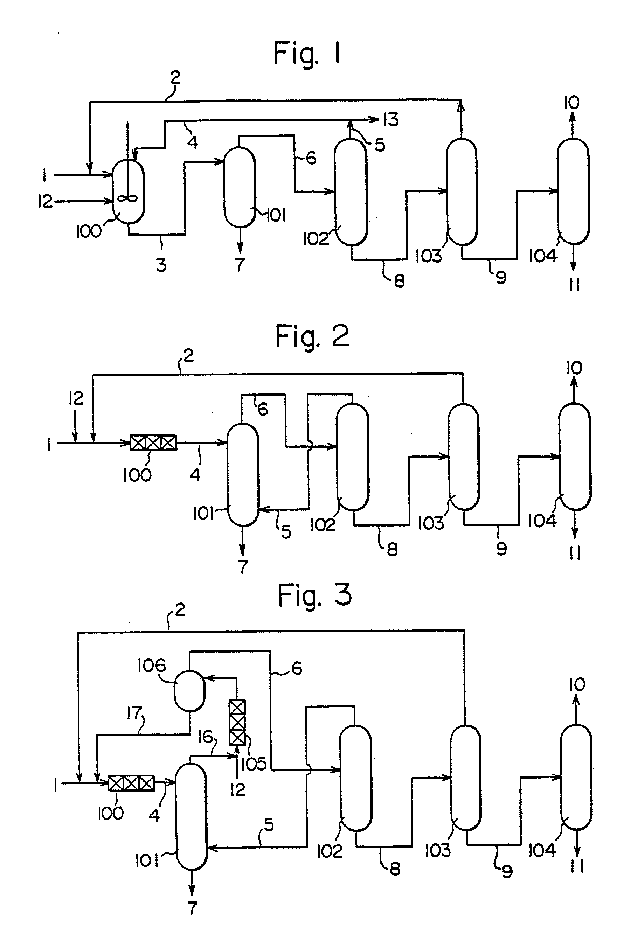

Figure 1 shows the purification of a crude MAA solution after mixing it with an aqueous bisulfite solution and an organic solvent using a continuous stirred vessel 100 as a mixer.

The crude MAA solution, a distillate from a low-boiling substance separating column 103, the aqueous bisulfite solution and as required, the organic solvent are fed into the stirred vessel 100 through lines 1, 2, 12, and 4, respectively. These materials are mixed with stirring, and the mixture is sent through a line 3 to a standing vessel 101 where it is separated into an aqueous layer and an organic layer. The aqueous layer is taken out through a line 7 and discharged out of the system. As required, useful components such as MAA and the organic solvent are recovered from the aqueous layer. The organic layer from a line 6 is fed to a solvent separating column 102. Low-boiling substances composed mainly of the solvent distill out from a line 5. They are partly recycled to the stirred vessel 100 through the line 4, and the remainder is taken out of the system through a line 13. The distillation bottom of the solvent separating column 102 is fed to the low-boiling substance separating column 103 through a line 8, and tiny amounts of low-boiling substances are recycled to the line 1 through the line 2. The distillation bottom in the low-boiling substance separating column 103 is sent to a high-boiling substance separating column (methacrylic acid rectifying column) 104 through a line 9, and MAA of high purity is obtained from the top of the column through a line 10. The distillation bottom in the column 104 is discharged through a line 11. It can be fed to a thin film evaporator, for example, in order to recover useful substances such as a polymerization inhibitor for MAA contained in the distillation bottom.

Figure 2 is a flow sheet of MAA purification which is applied to an aqueous solution of crude MAA and in which a line mixer 100 and an extracting column 101 are used instead of the stirred vessel 100 and the standing vessel 101 in Figure 1.

According to the method shown in Figure 2, an aqueous crude MAA solution obtained from the product gas of oxidation reaction, from which low-boiling substances such as methacrolein have been removed, the distillate of the low-boiling substance separating column 103, and an aqueous solution of a bisulfite are fed into the line mixer 100 through lines 1, 2 and 12, respectively, and mixed. The mixture is sent to the extracting column 101 through line 4. Preferably, a residence vessel where the mixture stays for about one hour is provided between the line 4 and the extracting column 101.

In the extracting column 101, the mixture is extracted with a solvent fed from a line 5. The extraction residue is discharged from line 7. Useful substances such as the solvent and acetic acid may be recovered from the extraction residue.

The extract is fed into the solvent separating column 102 through line 6. The solvent goes out from the top of the column and is sent to the extracting column 101 through the line 5 for reuse. The distillation bottom of the solvent separating column 102 is fed into the low-boiling substance separating column 103 through line 8, and the distillate mainly containing low-boiling substances is recycled to the extracting column 101. The distillation conditions are adjusted so that the amount of protoanemonin in the distillation bottom becomes less than 20 ppm, preferably less than 10 ppm. The distillation bottom is sent to the high-boiling substance separating column (methacrylic acid rectifying column) through line 9, and methacrylic acid of high purity is obtained from line 10 at the top of the column. The distillation bottom is discharged through line 11, but useful substances in the bottom such as methacrylic acid and the polymerization inhibitor can be recovered, for example, by using a thin film evaporator.

Figure 3 is a flow sheet showing a modification of the method shown in Figure 2, in which the extract is further washed with an aqueous solution of bisulfite.

According to the method shown in Figure 3, an aqueous solution of crude MAA obtained from the product gas of oxidation, from which low-boiling substances such as methacrolein have been removed, and an aqueous solution of bisulfite discharged from the bottom of an extract standing column (vessel) 106 are fed into line mixer 100 through lines 1 and 17, respectively, and fully mixed there. The mixture is then fed into the top of the extraction column 101 through line 4. Methacrylic acid is extracted with a solvent introduced from line 5 at the bottom, and the extraction residue is discharged through line 7. As required, the extraction residue is subjected to a step of recovering useful components such as the solvent or acetic acid. The extract from the top of the extraction column is sent to a line mixer 105 together with an aqueous solution of bisulfite from a line 12. They are kept in full contact with each other in the line mixer 105 to transfer a small amount of protoanemonin in the extract almost to the aqueous layer, and then the extract is sent to the extract standing column (vessel) 106. The solvent solution of methacrylic acid is sent to the solvent separating column through line 6. The solvent is distilled from the top of the column and is returned to the extraction column 101 through line 5. The distillation bottom is fed to the low-boiling substance separating column 103 through line 8, and from the top of the column, acrylic acid, acetic acid, etc. distill together with methacrylic acid. The distillate is fed into the line mixer 100 through line 2 together with the aqueous crude methacrylic acid from line 1. The distillation bottom of the low-boiling substance separating column 103 is fed into the high-boiling substance separating column (methacrylic acid rectifying column) 104 through line 9. Methacrylic acid is obtained from line 10, and the distillation bottom, from line 11.

Purified methacrylic acid obtained from a solution of crude methacrylic acid by the process of this invention contains less than 20 ppm of protoanemonin, and has superior polymerization characteristics.

The following examples illustrate the present invention more specifically.

Example 1

Using a molybdemum-type complex oxide as a first-stage reaction catalyst and a catalyst based on molybdenum-phosphorus type heteropolyacid as a second-stage reaction catalyst, isobutylene was catalytically oxidized with air in the presence of steam in the vapor phase. The reaction product gas was cooled and condensed using hydroquinone as a polymerization inhibitor. Low-boiling substances such as methacrolein were removed from the resulting aqueous solution containing methacrylic acid by distillation, and the residue was continuously extracted with toluene as an extracting solvent countercurrently to obtain an extract containing methacrylic acid. The extract was fed into a solvent separating column operated under reduced pressure to separate toluene. The resulting crude methacrylic acid solution contained 0.1% by weight of acetic acid, 0.14% by weight of acrylic acid, 30% by weight of toluene and 500 ppm by weight of protoanemonin as impurities.

The resulting crude methacrylic acid solution was purified in accordance with the flow sheet shown in Figure 1.

Ten kilograms of the crude methacrylic acid solution and 1 kg of a 23% by weight aqueous solution of sodium bisulfite were fed into the stirred vessel 100 through lines 1 and 12, respectively, and well mixed. The mixture was sent to the standing vessel 101 through line 3 and separated there into two layers. At this time, from line 7, 1.3 kg of the aqueous layer was discharged, and the concentration of methacrylic acid in this layer was 12% by weight. The organic layer was fed into the 10th tray of the solvent separating column 102 (inside diameter 2 inches; 30 sieve trays; made of SUS 304 stainless steel) through line 6, and distilled at a column top pressure of 75 mmHg and a reflux ration of 2.0. The solvent distilled out from the top of the column was recycled to the stirred vessel through lines 5 and 4. The distillation bottom contained 95.7% by weight of methacrylic acid. The distillation bottom was fed into the 15th tray of the low-boiling substance separating column 103 (inside diameter 2 inches; 40 sieve trays; made of SUS 304 stainless steel), and distilled under a column top pressure 35 mmHg at a reflux ratio of 20. The distillate containing low-boiling substances was recycled in an amount of 0.5 kg to the stirred vessel 100 through line 2. The recycle liquid contained 70.8% by weight of methacrylic acid, 20% by weight of toluene and 0.21% by weight of protoanemonin.

During the distillation for about 10 hours, no turbidity ascribable to polymerization was noted in the distillation bottom. As a polymerization inhibitor, phenothiazine was added to the distillation bottom in a concentration of 1000 ppm by weight. The distillation bottom was fed to the bottom of the high-boiling substance separating column 104 (inside diameter 2 inches; 15 sieve trays; made of SUS 304 stainless steel) through line 9, and rectified under a column top pressure of 35 mmHg at a reflux ratio of 0.5 to obtain 6.5 kg of purified methacrylic acid. The purified methacrylic acid contained 1 ppm by weight of protoanemonin, and its induction period of polymerization was 30 minutes. The induction period of polymerization was measured under the following conditions.

Sample: 10 cc of a 50% by weight aqueous solution of methacrylic acid (prepared from methacrylic acid and pure water)

Methoxyhydroquinone in methacrylic acid: 100 ppm by weight

Polymerization promoter: 2 cc of a 1% by weight aqueous solution of ammonium persulfate

Temperature: 70°C

## Example 2

By the same method as in Example 1, an aqueous solution of crude methacrylic acid from which low-boiling substances such as methacrolein had been removed was obtained. By using isobutyl acetate as an extracting solvent, the aqueous methacrylic acid solution was continuously extracted countercurrently to obtain an extract containing methacrylic acid. The extract was distilled under reduced pressure to separate isobutyl acetate. The resulting crude methacrylic acid contained 0.8% by weight of acetic acid, 0.25% by weight of acrylic acid, 400 ppm by weight of protoanemonin and 0.2% by weight of isobutyl acetate as impurities. It was purified by the same method and apparatus as used in Example 1.

Ten kilograms of the crude methacrylic acid and 2.17 kg of a 15% by weight aqueous solution of ammonium bisulfite were mixed in the stirred vessel, and at the same time, 6 kg of isobutyl acetate was added through line 4. After mixing with stirring, the mixture was separated into two layers in the standing vessel 101. At this time, 2.2 kg of an aqueous layer was obtained. It contained 9% by weight of methacrylic acid.

The organic layer was fed into the solvent separating column 102 where the solvent was recovered for reuse in the stirred vessel 100. The distillation bottom was continuously distilled for about 10 hours in the low-boiling substance separating column 103 in order to separate low-boiling substances. From line 9, 9.0 kg of the distillation bottom was obtained. No turbidity was observed in the distillation bottom. Phenothiazine had been added as a polymerization inhibitor to the distillation bottom in a concentration of 1000 ppm by weight. This distillation bottom was rectified at a reflux ratio of 0.5, and 8.6 kg of methacrylic acid of high purity was obtained from line 10.

The purified methacrylic acid contained 1.5 ppm by weight of protoanemonin, and its induction period of polymerization was 32 minutes.

## Comparative Example 1

Crude methacrylic acid was obtained by the same way as in Example 1. Without adding an aqueous solution of sodium bisulfite to the crude methacrylic acid solution, low-boiling substances such as the solvent were removed from it by distillation under reduced pressure in the same way as in Example 1. At this time, the amount of the distillation bottom from line 9 was 6.5 kg. In spite of the fact that phenothiazine had been added to the distillation bottom in a concentration of 2000 ppm by weight, the distillation bottom was turbid. The distillation bottom was distilled under the same conditions using the same apparatus as in Example 1. When 4.2 kg of the distillate was obtained from line 10, scales attributed to a polymer adhered to the still portion. Hence, the distillation was stopped. The resulting methacrylic acid contained 60 ppm by weight of protoanemonin, and its induction period of polymerisation was 40 minutes.

Example 3

Using a molybdenum-type complex oxide as a first-stage reaction catalyst and a catalyst based on molybdenum-phosphorus type heteropolyacid as a second-stage reaction catalyst, isobutylene was catalytically oxidized with air in the vapor phase in the presence of steam. The reaction product gas was cooled and condensed using hydroquinone as a polymerization inhibitor to obtain an aqueous solution containing methacrylic acid. Low-boiling substances such as methacrolein were removed from the resulting aqueous solution by distillation to give 20 kg/hr of an aqueous solution containing 24% by weight of methacrylic acid, 3.6% by weight of acetic acid, 1.4% by weight of phthalic acids (o-, m-, p-), 0.8% by weight of maleic acid, 1.0% by weight of tarry substances and 100 ppm of protoanemonin. This aqueous solution of crude methacrylic acid was purified in accordance with the flow sheet shown in Figure 2.

The methacrylic acid aqueous solution (20 kg/hr) and a 30% aqueous solution of sodium bisulfite (0.5 kg/hr) were fed into the line mixer 100 through lines 1 and 12, respectively, and they were mixed there. The mixture was fed into the top of the extraction column through line 4. From the bottom of the extraction column 101, toluene was fed at a rate of 20 kg/hr through line 5 to extract it continuously in a countercurrent fashion. The extracting operation was carried out at room temperature and atmospheric pressure. The extraction column was a rotating disc-shaped column having an inside diameter of 70 mm and a total height of 1800 mm. After the extraction was fully caused to reach equilibrium, the extract (organic layer) was obtained from line 6 at a rate of 26 kg/hr. From line 7, the extraction residue (aqueous layer) was obtained at a rate of 14.5 kg/hr. The amount of protoanemonin in the extract was 24 ppm, and the amount of protoanemonin in the extraction residue was 135 ppm. The resulting extract was fed to the 15th tray of the solvent separating column 102 (inside diameter 6 inches; 30 sieve trays; made of SUS 304 stainless steel), and distilled under a column top pressure of 75 mmHg at a reflux ration of 1.0 (heated by a steam reboiler at 2 kg/cm²—G). The solvent which distilled from the top of the column was recycled to the extraction column 101 through line 5 for reuse. As a distillation bottom, 98.2% by weight of methacrylic acid was obtained from line 8. The distillation bottom was fed into the 15th tray of the low-boiling substance separating column 103 (inside diameter 6 inches; 40 sieve trays; made of SUS 304 stainless steel) through line 8 and distilled under a column top pressure of 35 mmHg at a reflux ratio of 15 (heated by a steam reboiler at 2 kg/cm²—G). The distillate containing low-boiling substances was recycled to the extraction column 101 at a rate of 0.4 kg/hr. The recycled liquid contained 83.5% by weight of methacrylic acid and 0.12% by weight of protoanemonin. The distillation bottom was fed into the bottom of the high-boiling substance separating column 104 (inside diameter 6 inches; 15 sieve trays; made of SUS 304 stainless steel) through line 9, and distilled under a column top pressure of 35 mmHg at a reflux ratio of 2.0 (heated by a steam reboiler under 2 kg/cm²—G). Purified methacrylic acid was obtained from line 10. Gas-chromatographic analysis showed that the purified methacrylic acid had a purity of more than 99.9% by weight and contained 8 ppm of protoanemonin.

Comparative Example 2

The same aqueous methacrylic acid solution as used in Example 3 was extracted by the same method and apparatus as used in Example 1 except that sodium bisulfite was not added. Solids precipitated on the interface in the extraction column, but the operation was continued while occasionally discharging the solids. After an extraction equilibrium was reached, 25.8 kg/hr of an extract and 14.2 kg/hr of an extraction residue were obtained. The extract contained 80 ppm of protoanemonin, and the extraction residue contained 46 ppm of protoanemonin. The resulting extract was subjected to the same purifying treatment as in Example 3. But methacrylic acid obtained from line 10 contained 280 ppm of protoanemonin. The distillation bottom in the low-boiling substance separating column was turbid.

Example 4

The same aqueous methacrylic acid solution as used in Example 3 was extracted by the same method and apparatus as in Example 3 except that a 30% by weight aqueous solution of ammonium bisulfite was fed at a rate of 1.0 kg/hr through line 12 instead of sodium bisulfite. The mixture obtained in the line mixer was fed into the extraction column 101 through line 4. After an extraction equilibrium was fully reached, an extract was obtained at a rate of 26 kg/hr from line 6, and an extraction residue, at a rate of 15 kg/hr from line 7. The extract contained 25 ppm of protoanemonin, and the extraction residue contained 130 ppm of protoaneminin. The extract was purified in the same way as in Example 3 except that the reflux ratio in the operation of the low-boiling substance separating column 103 was changed to 25. Purified methacrylic acid obtained from line 10 contained 10 ppm of protoanemonin.

Example 5

Using a molybdenum-type complex oxide as a first-stage reaction catalyst and a molybdenum-phosphorus type heteropolyacid as a second-stage reaction catalyst, isobutylene was catalytically oxidized with air in the vapor phase in the

presence of steam. The reaction product gas was cooled and condensed using hydroquinone as a polymerization inhibitor to obtain an aqueous solution containing methacrylic acid. Low-boiling substances such as methacrolein were removed from the resulting aqueous solution by distillation to obtain 20 kg/hr of an aqueous solution containing 24% by weight of methacrylic acid, 3.6% by weight of acetic acid, 1.4% by weight of phthalic acids (o-, m-, p-), 0.8% by weight of maleic acid, 1.0% by weight of tarry substances and 100 ppm of protoanemonin. This aqueous solution was purified in accordance with the flow sheet shown in Figure 3.

A 30% by weight aqueous solution of sodium bisulfite (0.5 kg/hr) and an extract (26 kg/hr) from the top of the extraction column were fed to the line mixer through lines 12 and 16, respectively, and mixed. The mixture was fed into the standing vessel 106. The aqueous layer at the lower part was mixed with an aqueous methacrylic acid solution (20 kg/hr) fed through line 1 by the line mixer 100. The mixture was fed into the extraction column 101 through line 4. Isobutyl acetate as a solvent was fed into the bottom of the extraction column 101 at a rate of 20 kg/hr through line 5 to extract the aqueous methacrylic acid solution continuously in a countercurrent fashion. The extracting operation was carried out at room temperature and atmospheric pressure. The extraction column was a rotary disc-shaped column having an inside diameter of 70 mm and a total height of 1800 mm. After an extraction equilibrium was fully reached, an extract (organic layer) was obtained at a rate of 26 kg/hr through line 6 via the standing vessel 106, and an extraction residue (aqueous layer) was obtained at a rate of 14.6 kg/hr through line 7. The extract from line 6 contained 3 ppm of protoanemonin, and the extraction residue from line 7 contained 134 ppm of protoanemonin. The resulting extract was fed into the 15th tray of the solvent separating column 102 (inside diameter 6 inches; 30 sieve trays; made of SUS 304 stainless steel) through line 6, and distilled under a column top pressure of 75 mmHg at a reflux ratio of 1.0 (heated by a steam reboiler under 2 kg/cm$^2$—G). The solvent which distilled from the top of the column was recycled to the extraction column 101 through line 5 for reuse. The distillation bottom obtained from line 8 contained 88.0% by weight of methacrylic acid, 9.1% by weight of acetic acid and 1.4% by weight of acrylic acid. The distillation bottom was fed into the 15th tray of the low-boiling substance separating column 103 (inside diameter 6 inches; 40 sieve trays; made of SUS 304 stainless steel), and distilled under a column top pressure of 35 mmHg at a reflux ratio of 15 (heated by a steam reboiler under 2 kg/cm$^2$—G). From the top of the column, a distillate containing 56.0% by weight of methacrylic acid, 36.4% by weight of acetic acid and 5.6% by weight of acrylic acid was obtained. This distillate was recycled to the line mixer 100 through line 2. The distillation bottom in the column 103 was fed into the bottom of the high-boiling substance separating column 104 (inside diameter 6 inches; 15 sieve trays; made of SUS 304 stainless steel) through line 9, and distilled under a column top pressure of 35 mmHg at a reflux ratio of 2.0 to obtain purified methacrylic acid from line 10. Gas-chromatographic analysis showed that this product had a purity of more than 99.9% by weight, and contained 7 ppm of protoanemonin.

Comparative Example 3

The same aqueous methacrylic acid solution as used in Example 5 was subjected to an extracting treatment by the same method and apparatus as in Example 5 except that sodium bisulfite was not added. Solids precipitated on the interface of the extraction column. But the operation was continued while occasionally removing the solids. After an extraction equilibrium was reached, 25.8 kg/hr of an extract and 14.2 kg/hr of an extraction residue were obtained, respectively. The extract contained 69 ppm of protoanemonin, and the extraction residue contained 15 ppm of protoanemonin. The resulting extract was subjected to the same purifying treatment as in Example 5. Methacrylic acid obtained from line 10 contained 310 ppm of protoanemonin, and the distillation bottom of the low-boiling substance separating column 103 was turbid.

Example 6

The same aqueous methacrylic acid solution as used in Example 5 extracted by the same method and apparatus as in Example 5 except that instead of sodium bisulfite from line 12, a 30% by weight aqueous solution of ammonium bisulfite ws used at a rate of 1.0 kg/hr, and mixed well with the extract from line 16 in the line mixer 105, and the mixture was fed into the standing vessel 106. After an extraction equilibrium was fully reached, an extract (26 kg/hr) and an extraction residue (15 kg/hr) were obtained through lines 6 and 7, respectively. The extract contained 3.5 ppm of protoanemonin, and the extraction residue contained 133 ppm of protoanemonin. The resulting extract was purified in the same way as in Example 5. Purified methacrylic acid obtained from line 10 contained 11 ppm of protoanemonin.

**Claims**

1. In the production of methacrylic acid by catalytically oxidizing isobutylene, tertiary butanol, methacrolein or isobutyraldehyde with a molecular oxygen-containing gas in the vapor phase, a process for purifying methacrylic acid which comprises bringing a solution of crude methacrylic acid obtained from the reaction product gas of said oxidation into contact with an aqueous solution of a bisulfite in the presence of a substantially water-insoluble organic solvent to separate it into an organic layer and an aqueous layer, feeding the organic layer into a solvent separating column and distilling off the solvent mainly, feeding the distillation bottom of the solvent separating column into a low-boiling substance separating column and distilling off low-boiling substances, mixing the low-boiling substances with said crude methacrylic acid solution, and feeding the distillation bottom of the low-boiling substance separating column into a high-boiling substance separating column and recovering methacrylic acid of high purity as a distillate.

2. The process of claim 1 wherein said crude methacrylic acid solution is an aqueous solution of methacrylic acid obtained by cooling the reaction product gas.

3. The process of claim 1 wherein said crude methacrylic acid solution is a mixture consisting mainly of a substantially water-insoluble organic solvent and methacrylic acid which is obtained by subjecting an aqueous solution of methacrylic acid obtained by cooling the reaction product gas to an extraction operation with the organic solvent.

4. The process of claim 1 wherein the resulting organic layer is again contacted with an aqueous solution of a bisulfite.

**Patentansprüche**

1. Verfahren zur Reinigung von Methacrylsäure, welche durch katalytische Oxidation von Isobutylen, tert. Butanol, Methacrolein oder Isobutyraldehyd mit einem molekularen Sauerstoff enthaltenden Gas in der Dampfphase hergestellt worden ist, dadurch gekennzeichnet, daß man eine Lösung der aus dem Reaktionsproduktgas der genannten Oxidation erhaltenen rohen Methacrylsäure mit einer wäßrigen Bisulfitlösung in Gegenwart eines im wesentlichen wasserunlöslichen organischen Lösungsmittels kontaktiert, um sie in eine organische und eine wäßrige Schicht aufzutrennen, daß man die organische Schicht in eine Lösungsmittel-Trennsäule einspeist und das Lösungsmittel weitgehend abdestilliert, daß man den Destillationsrückstand der Lösungsmittel-Trennsäule in eine Trennsäule für niedrigsiedende Substanzen einspeist und die niedrigsiedenden Substanzen abdestilliert, daß man die niedrigsiedenden Substanzen mit der genannten rohen Methacrylsäurelösung vermischt und daß man den Destillationsrückstand der Trennsäule für niedrigsiedende Substanzen in eine Trennsäule für hochsiedende Substanzen einspeist und Methacrylsäure von hoher Reinheit als Destillat gewinnt.

2. Verfahren nach Anspruch 1, dadurch gekennzeichnet, daß die rohe Methacrylsäurelösung eine wäßrige Lösung von Methacrylsäure ist, die durch Abkühlung des Reaktionsproduktgases erhalten worden ist.

3. Verfahren nach Anspruch 1, dadurch gekennzeichnet, daß die rohe Methacrylsäurelösung ein Gemisch ist, das hauptsächlich aus einem im wesentlichen wasserunlöslichen organischen Lösungsmittel und Methacrylsäure erhalten durch Extraktion einer durch Abkühlung des Reaktionsproduktgases erhaltenen wäßrigen Lösung von Methacrylsäure mit einem organischen Lösungsmittel, besteht.

4. Verfahren nach Anspruch 1, dadurch gekennzeichnet, daß man die erhaltene organische Phase erneut mit einer wäßrigen Bisulfitlösung kontaktiert.

**Revendications**

1. Dans la production d'acide méthacrylique par oxydation catalytique en phase vapeur d'isobutylène, de tertio-butanol, de méthacroléine ou d'isobutyraldéhyde avec un gaz contenant de l'oxygène moléculaire, un procédé de purification d'acide méthacrylique qui consiste à amener une solution d'acide méthacrylique brut obtenue à partir du produit réactionnel gazeux de ladite oxydation en contact avec une solution aqueuse d'un bisulfite en présence d'un solvant organique sensiblement insoluble dans l'eau pour la séparer en une phase organique et une phase aqueuse, à introduire la phase organique dans une colonne de séparation du solvant et chasser par distillation principalement le solvant, à introduire le résidu de distillation de la colonne de séparation du solvant dans une colonne de séparation des substances de bas point d'ébullition et chasser par distillation les substances de bas point d'ébullition, à mélanger les substances de bas point d'ébullition avec ladite solution d'acide méthacrylique brut, et à introduire le résidu de distillation de la colonne de séparation des substances de bas point d'ébullition dans une colonne de séparation des substances de haut point d'ébullition et recueillir de l'acide méthacrylique de grande pureté en tant que distillat.

2. Procédé selon la revendication 1 dans lequel ladite solution d'acide méthacrylique brut est une solution aqueuse d'acide méthacrylique obtenue par refroidissement du produit réactionnel gazeux.

3. Procédé selon la revendication 1 dans lequel ladite solution d'acide méthacrylique brut est un mélange consistant principalement en un solvant organique sensiblement insoluble dans l'eau et en acide méthacrylique, qui est obtenu en soumettant une solution aqueuse d'acide méthacrylique obtenue par refroidissement du produit réactionnel gazeux à une opération d'extraction avec le solvant organique.

4. Procédé selon la revendication 1 dans lequel la phase organique résultante est de nouveau mise en contact avec une solution aqueuse d'un bisulfite.

# 0 102 642

## Fig. 1

## Fig. 2

## Fig. 3

1